# EUROPEAN PATENT SPECIFICATION

(11) **EP 1 423 012 B1**
(45) Date of publication and mention of the grant of the patent: **14.11.2007**
(21) Application number: 02752822.3
(22) Date of filing: 12.08.2002
(51) Int. Cl.: A61K 35/28, A61P 9/00, A61P 19/00, A61P 21/00, A61P 25/00, C12N 5/06

(54) **MEDICAL USE OF STEM CELLS EXPRESSING VEGFR-1**
MEDIZINISCHE VERWENDUNG VON STAMMZELLEN, DIE VEGFR-1 EXPRIMIEREN
UTILISATION MEDICALE DE CELLULES SOUCHES EXPRIMANT VEGFR-1

(30) Priority: 10.08.2001 US 311705 P
(43) Date of publication of application: 02.06.2004
(73) Proprietor: IMCLONE SYSTEMS, INC., New York, NY 10014 (US); Cornell Research Foundation Inc., Ithaca, NY 14850 (US)
(72) Inventor: RAFII, Shahin, Great Neck, NY 11021 (US); WITTE, Larry, Stormville, NY 12582 (US)
(74) Representative: Beetz & Partner
(86) International application number: PCT/US2002/025657
(87) International publication number: WO 2003/014326

(56) References cited:
- EP-A- 1 199 565
- WO-A-01/56593
- WO-A-01/76620
- BÜHRING H J ET AL: "Expression of novel surface antigens on early hematopoietic cells." ANNALS OF THE NEW YORK ACADEMY OF SCIENCES. 30 APR 1999, vol. 872, 30 April 1999 (1999-04-30), pages 25-38 ; disc, XP009039160 ISSN: 0077-8923
- DATABASE BIOSIS [Online] BIOSCIENCES INFORMATION SERVICE, PHILADELPHIA, PA, US; 16 November 2000 (2000-11-16), GILL M T ET AL: "Cytokine induced proliferation and maturation of hematopoietic progenitor cells promote expansion of VEGFR-1 positive cells" XP002303839 Database accession no. PREV200100311709
- SAWANO A ET AL: "Flt-1, vascular endothelial growth factor receptor 1, is a novel cell surface marker for the lineage of monocyte-macrophages in humans" BLOOD, W.B.SAUNDERS COMPAGNY, ORLANDO, FL, US, vol. 97, no. 3, 1 February 2001 (2001-02-01), pages 785-791, XP002297914 ISSN: 0006-4971
- GALLACHER LISA ET AL: "Isolation and characterization of human CD34-Lin- and CD34+Lin- hematopoietic stem cells using cell surface markers AC133 and CD7" BLOOD, vol. 95, no. 9, 1 May 2000 (2000-05-01), pages 2813-2820, XP002303837 ISSN: 0006-4971
- PASINO MIRELLA ET AL: "Flow cytometric and functional characterization of AC133+ cells from human umbilical cord blood" BRITISH JOURNAL OF HAEMATOLOGY, vol. 108, no. 4, March 2000 (2000-03), pages 793-800, XP002303838 ISSN: 0007-1048
- TSENG-LAW JANET ET AL: "Identification of a peptide directed against the anti-CD34 antibody, 9C5, by phage display and its use in hematopoietic stem cell selection" EXPERIMENTAL HEMATOLOGY, NEW YORK, NY, US, vol. 27, no. 5, May 1999 (1999-05), pages 936-945, XP002192619 ISSN: 0301-472X
- CUMANO A ET AL: "Pluripotent hematopoietic stem cell development during embryogenesis" CURRENT OPINION IN IMMUNOLOGY, CURRENT BIOLOGY LTD, XX, vol. 13, no. 2, 1 April 2001 (2001-04-01), pages 166-171, XP004257780 ISSN: 0952-7915
- PEICHEV MM ET AL: "Expression of VEGFR-2 and AC133 by circulating human CD34+ cells identifies a population of functional endothelial precursors" BLOOD, W.B.SAUNDERS COMPAGNY, ORLANDO, FL, US, vol. 95, no. 3, 1 February 2000 (2000-02-01), pages 952-958, XP002251073 ISSN: 0006-4971
- ROSSIGNOL M ET AL: "Genomic Organization of Human Neuropilin-1 and Neuropilin-2 Genes: Identification and Distribution of Splice Variants and Soluble Isoforms" GENOMICS, ACADEMIC PRESS, SAN DIEGO, US, vol. 70, no. 2, 1 December 2000 (2000-12-01), pages 211-222, XP004437754 ISSN: 0888-7543
- GEHLING U M ET AL: "In vitro differentiation of endothelial cells from AC133-positive progenitor cells" BLOOD, W.B.SAUNDERS COMPAGNY, ORLANDO, FL, US, vol. 95, no. 10, 15 May 2000 (2000-05-15), pages 3106-3112, XP002254113 ISSN: 0006-4971
- PLATE K.H.: 'Mechanisms of angiogenesis in the brain' JOURNAL OF NEUROPATHOLOGY AND EXPERIMENTAL NEUROLOGY vol. 58, no. 4, April 1999, pages 313 - 320, XP002202421
- MANDRIOTA S.J. ET AL.: 'Vascular endothelial growth factor-induced in vitro angiogenesis and plasminogen activator expression are dependent on endogenous basic fibroblast growth factor' JOURNAL OF CELL SCIENCE vol. 110, 1997, pages 2293 - 2302, XP002960932
- HORNIG C. ET AL.: 'Detection and quantification of complexed and free soluble human vascular endothelial growth factor receptor-1 (sVEGFR-1) by ELISA' JOURNAL OF IMMUNOLOGICAL METHODS vol. 226, 1999, pages 169 - 177, XP004171370
- CORTES F. ET AL.: 'Differential expression of KDR/VEGFR-2 and CD34 during mesoderm development of the early human embryo' MECHANISMS OF DEVELOPMENT vol. 83, 1999, pages 161 - 164, XP002960933
- KHALIQ A. ET AL.: 'Hypoxia down-regulates placenta growth factor, whereas fetal growth restriction up-regulates placental growth factor expression: molecular evidence for "placental hyperoxia" in intrauterine growth restriction' LABORATORY INVESTIGATION vol. 79, no. 2, 1999, pages 151 - 170, XP002960931

## Description

### FIELD OF THE INVENTION

The present invention is directed to methods of isolating and mobilizing mammalian stem cells expressing vascular endothelial growth factor (VEGF) receptor 1 (VEGFR-1), also known as *fms*-like tyrosine kinase receptor-1 (FLT-1), and to the pharmaceutical use of such stem cells.

### BACKGROUND OF THE INVENTION

Stem cells are unique cell populations with the ability to undergo both self-renewal and differentiation. In mammalian embryos, hemangioblasts are believed to be the precursors of angioblasts and totipotent or pluripotent hematopoietic stem cells. Angioblasts and other embryonic totipotent and/or pluripotent stem cells are believed to be the precursors of postnatal endothelial cells, muscle cells, and neural cells.

The mammalian hematopoietic system comprises erythrocytes (red blood cells) and white blood cells that mature from more primitive lineages. *See, e.g.,* U.S. Patent Nos. 5,747,651 and 5,912,133 (referencing Dexter and Spooncer, Ann. Rev. Cell Biol., 3: 423-441 (1987)).

The erythrocytes result from primitive cells called erythroid burst-forming units, whose immediate progeny are called erythroid colony-forming units. The white blood cells contain the mature cells of the lymphoid and myeloid systems. The lymphoid cells include B lymphocytes and T lymphocytes, both of which result from earlier progenitor cells (Dexter and Spooncer). The myeloid system comprises a number of cells including granulocytes, platelets, monocytes, macrophages and megakaryocytes. The granulocytes are further divided into neutrophils, eosinophils, basophils, and mast cells. Each of the mature hematopoietic cells is specialized for specific functions.

The development of the initial blood vessel system in embryos is generally believed to occur from the adhesion to each other and modeling of primitive endothelial precursor cells, such as angioblasts. This process is generally known as vasculogenesis.

Postnatal development of new blood vessels is generally believed to occur from the proliferation, migration, and remodeling of the mature endothelial cells of pre-existing, blood vessels. This process is generally known as angiogenesis.

One or more totipotent stem cells can undergo a series of differentiation steps leading to increasingly lineage-restricted progenitor cells. For example, a totipotent stem cell of a certain cell type, e.g. a hematopoietic stem cell, an endothelial stem cell, a muscle stem cell or a neural stem cell, is capable of reconstituting all cells of that cell type *in vivo.* The more mature stem cells have limited proliferative capacity and are generally capable of giving rise to only one or two lineages *in vitro* or *in vivo*.

Stem cell-base therapies have a broad variety of applications. For example, stem cell induction of hematopoiesis, vasculogenesis and/or angiogenesis, myogenesis and neurogenesis can be employed to treat various conditions. Thus, stem cells can be used to treat diseases that result from the destruction and/or dysfunction of a limited number of cell types, such as diabetes mellitus, in which pancreatic islet cells have been selectively destroyed, or Parkinson's disease, which results from the destruction of dopaminergic neurons within a particular region of the brain. Other applications include treatment of, for example, peripheral ischemia, sickle cell anemia, thalassemia, muscular dystrophy, Alzheimer's disease, traumatic spinal cord injury, Purkinje cell degeneration, liver failure, cardiac ischemia, Duchenne's muscular dystrophy, and osteogenesis imperfecta, as well as in combination with chemotherapy or radiation treatments. A human stem cell-based strategy could also be employed to generate an unlimited supply of cells or tissue from an abundant, renewable, and readily accessible source for use in organ transplants. Moreover, by virtue of their permissiveness for stable genetic modification, stem cells could be engineered to escape or inhibit host immune responses. Moreover, stem cells have various applications in basic research, including studies relating to developmental biology.

Although stem cell-based therapy holds great promise to successfully treat a variety of diseases, many barriers remain. One such barrier involves the isolation of purified populations of stem cells. In this regard, efforts have been made to use various surface markers to obtain purified populations of stem cells. For example, a purified population of CD34+ hematopoietic stem cells was described by Civin in U.S. Patent No. 5,035,994 and U.S. Patent No. 5,130,144. A more highly purified population of hematopoietic stem cells that are CD34+, Class II HLA+, and Thy-1+ was described by Tsukamoto et al. in U.S. Patent No. 5,061,620. The Tsukamoto patent further explains that stem cells lack certain markers that are characteristic of more mature, lineage-committed (Lin+) cells. Such markers include CD3, CD8, CD10, CD19, CD20, and CD33. Cells that lack these markers are said to be lineage negative (Lin-).

In addition, it is known that growth factors play an important role in the development and operation of mammalian stem cells. The role of these growth factors is complex. For example, hematopoiesis can be established in the absence of growth factors, provided that marrow stromal cells are added to the medium.

Hematopoietic growth factors exhibit a spectrum of activities. For instance, erythropoietin is believed to promote proliferation of only mature erythroid progenitor cells. IL-3, which is believed to facilitate the growth and development of early stem cells as well as of numerous progenitor cells, including those restricted to the granulocyte/macrophage, eosinophil, megakaryocyte, erythrocyte and mast cell lineages. Another hematopoietic growth factor whose receptor is the product of the W locus, c-kit, is a member of the class of receptor protein tyrosine kinases (pTK). *See, e.g.,* Anderson et al., Cell, 63(1): 235-43 (1990); Huang et al., Cell, 63(1): 225-33 (1990); Zsebo et al., Cell, 63(1): 213-24 (1990); Zsebo et al., Cell, 63(1): 195-201 (1990); Flanagan & Leder, Cell, 63(1): 195-94 (1990); Copeland et al., Cell, 63(1): 175-85 (1990); Williams et al., Cell, 63(1): I64-74 (1990). The ligand for c-kit, referred to by various names, e.g. stem cell factor (SCF) and mast cell growth factor (MGF) is believed to be essential for the development of early hematopoietic stem cells and cells restricted to the erythroid and mast cell lineages in mice. *Id*.

It is becoming increasingly apparent that these protein tyrosine kinases (pTK) also play an important role as cellular receptors for stem cell growth factors. The pTK family has several conserved amino acid regions in the catalytic domain (see e.g. Hanks et al., Science, 241:42-52 9 (1988); Wilks, PNAS USA, 86:1603-1607 (1989)). Other specific examples of protein tyrosine kinases include the vascular endothelial growth factor (VEGF) receptors. There are two such receptors, *fms*-like tyrosine kinase (FLT-1), also known as VEGFR-1, which was sequenced by Shibuya et al., Oncogene, 5: 519-524 (1990), and kinase insert domain-containing receptor/fetal liver kinase (KDR/flk-1), also known as VEGFR-2, which was described in WO 92/14248, filed February 20, 1992, and Terman et al., Oncogene, 6: 1677-1683 (1991), and sequenced by Matthews et al., PNAS USA, 88: 9026-9030 (1991).

It is generally believed that VEGFR-2 is the main signal transducer for VEGF, resulting in endothelial cell proliferation, migration, differentiation, tube formation, increase of vascular permeability, and maintenance of vascular integrity. VEGFR-1 possesses a much weaker kinase activity, and is unable to generate a mitogenic response when stimulated by VEGF - although it binds to VEGF with an affinity that is approximately 10-fold higher than VEGFR-2. VEGFR-1 has also been implicated in VEGF and placenta growth factor (P1GF) -induced migration of monocytes and macrophages, and production of tissue factors.

The VEGF homologue P1GF is also a natural specific ligand for VEGFR-1. PIGF, a dimeric secreted factor, is produced in large amounts by villous cytotrophoblast, sincytiotrophoblast and extravillous trophoblast and has close amino acid homology to VEGF. Three isoforms exist in humans, PIGF-1, P1GF-2, and P1GF-3. Studies with PIGF-deficient mice demonstrate that this growth factor is not involved in angiogenesis *per se,* but rather, specifically modulates the angiogenic and permeability effects of VEGF during pathological situations.

The publication of Bühring, H.-J., et al. "Expression of novel surface antigens on early hematopoietic cells", ANNALS OF THE NEW YORK ACADEMY OF SCIENCES, vol. 872, 30 April 1999, pages 25-38, relates to the determination of the surface marker profile of hematopoietic stem cells (bone marrow cells, BM cells) and monoclonal antibodies against cell surface antigens of those stem cells. These antibodies have been used for selectively recognizing immature hematopoietic cells and for further dissecting populations within the BM stem cell compartment. This article specifically describes the expression of AC 133 or CD34⁺ and CD34⁺CD38⁻ human BM cells and the coexpression patterns of angiopoietin receptors TIE and TEK, the vascular endothelial growth factor (VEGF) receptor FLT1, FLT4 and KDR, and HER-2 and FLT3. It is stated in this document (page 26) that the FLT1 receptors are only expressed at very low copy numbers (7%) on the cell surface. The document does not comprise any description of isolating such cells expressing VEGFR-1 (FLT1); the cells have only been detected, and their presence has been determined using a novel magneto-fluorescent liposome staining technique to increase the sensitivity of immunofluorescence. Further, there is no description of using VEGFR-1 expressing stem cells as such or in the form of compositions for the preparation of medicaments or any suggestion that such particular cells could be used for medical treatment.

The publication of Gill, M.T., et al., "Cytokine induced proliferation and maturation of hematopoietic progenitor cells promote expansion of VEGFR-1 positive cells". Database Biosis (Online) BIOSCIENCES INFORMATION SERVICE, PHILADELPHIA, PA, US, 16 November 2000, Database accession No. PREV200100311709, Abstract, relates to the cytokine-induced proliferation and maturation of hematopoietic progenitor cells which promote expansion of VEGFR-1 positive cells. It has been shown that VEGFR-1 is expressed on endothelial as well as myeloid cells. Using monoclonal antibodies to VEGFR-1 it was demonstrated that VEGFR-1 is expressed on 2.4±0.8 % AC133⁺ cells derived from human fetal liver. This article does not describe isolating such cells, and there is no description of using VEGFR-1 expressing stem cells for the preparation of medicaments, nor any suggestion that such stem cells could be used for such purposes.

The article of L. Gallacher et al., "Isolation and characterization of human CD34-Lin- and CD23+Lin- hematopoietic stem cells using stem cell surface markers AC133 and CD7", Blood 95, No. 9, 1 May 2000, pages 2813-2819, deals with isolation and characterization of human CD34⁻Lin⁻ and CD34⁺Lin⁻ stem cells expressing AC133 or CD7. In the introduction part of this publication, the utility of human stem cells for gene therapy, correction of genetic disorders, transplantation for purposes of haematological recovery and long-term engraftment is mentioned only in a very generic manner, without reference to any concrete stem cell type to be used for these purposes. There is no disclosure of stem cells expressing VEGFR-1.

The article of M. Pasino et al., "Flow cytometric and functional characterization of AC133+ cells from human umbilical cord blood", British Journal of Haematology 2000, 108, 793-800, relates to the characterization of AC133⁺ stem cells from human umbilical cord blood (UCB) and the comparison of their immunophenotypic and functional features with those of UCB CD34⁺ cells. In this article there is no mention of any utility of the characterized AC133⁺ UCB cells, nor any mention of stem cells expressing VEGFR-1.

It is the underlying problem of the present invention to provide a method for the selective isolation of mammalian stem cells expressing VEGFR-1 from a stem cell population, and to use those stem cells for specific therapeutic purposes.

The above problem is solved according to claim 1. The dependent claims relate to preferred embodiments.
The invention teaches the use of mammalian stem cells expressing VEGFR-1 or of compositions comprising mammalian stem cells expressing VEGFR-1 for preparing pharmaceutical compositions for the induction of hematopoiesis, vasculogenesis, angiogenesis, myogenesis, neurogenesis, mobilization of stem cells to the area of administration or for reconstituting pancreatic islet cells, regenerating damaged neurons or destructed bone marrow or for the treatment of cardiac or peripheral ischemia,
wherein the stem cells are no human embryonic stem cells, and are obtainable by
- providing a population of cells,
- contacting the population of cells with a molecule that specifically binds to VEGFR-1, and
- isolating cells that bind the molecule that specifically binds to VEGFR-1.

### SUMMARY OF THE INVENTION

The present invention provides methods of isolating mammalian stem cells expressing the VEGF receptor VEGFR-1 and of compositions comprising mammalian stem cells expressing VEGFR-1. The isolated cells preferably include hematopoietic stem cells, endothelial stem cells, muscle stem cells and neural stem cells, which are preferably human stem cells. In a preferred embodiment, the present invention provides methods of further positively and/or negatively selecting for stem cells, Also provided are methods of using such isolated mammalian stem cells expressing VEGFR-1 to treat various conditions, which can involve inducing hematopoiesis, vasculogenesis and/or angiogenesis, myogenesis, and neurogenesis to treat the various condition. Molecules such as P1GF can stimulate proliferation and/or differentiation and mobilization, i.e., motogenesis, of stem cells, which can then be employed to treat various conditions.

This technology also allows for mobilizing a large number of stem cells to the peripheral circulation. The enriched population of the stem cells in the peripheral circulation facilitates isolation of large numbers of stem cells expressing VEGFR-1 that can be used for gene therapy or bone marrow transplantation. In addition, these stem cells can directly or intravenously be used for restoring function to the ischemic myocardium, reconstitute pancreatic islet cells, or regenerate damaged neurons.

### BRIEF DESCRIPTION OF THE FIGURES

**Figure 1** is a graph of the survival (%) as a function of time (days) of lethally irradiated mice after administration of varying concentrations (10⁵, 10³, 10², and 10) of VEGFR-1 positive cells or VEGFR-1 negative cells (10⁵).

**Figure 2** is a graph of the survival (%) as a function of time (days) of lethally irradiated mice after administration of VEGFR-1 positive and/or Sca-1 positive cells.

**Figure 3** is a graph of survival (%) as a function of time (days) of mice treated with a sub-lethal dose of 5 fluorouracil (5FU) after administration of neutralizing monoclonal antibodies to VEGFR-1 or VEGFR-2.

**Figure 4** is a graph of the number of white blood cells (WBC) (µl) as a function of time (days) in mice treated with a sub-lethal dose of 5FU after administration of an adenoviral vector expressing P1GF.

**Figure 5** is a graph of the number of WBC (µl) as a function of time (days) in mice treated with carboplatin plus total body irradiation (TBI) after administration of an adenoviral vector expressing P1GF.

**Figure 6** is a graph of the number of migrated cells (x 10³ cells) in transmigration Boyden Chambers after administration of P1GF, PIGF and monoclonal antibodies to VEGFR-1, VEGF, VEGF and monoclonal antibodies to VEGFR-1, or SDF-1.

**Figures 7A-D** are graphs of the number of colonies in the peripheral blood of lethally irradiated syngeneic mice after administration of P1GF at day 1 (Fig. 7A), day 3 (Fig. 7B), day 10 (Fig. 7C), and day 14 (Fig. 7D).

**Figure 8** is a graph of the number of CFU-S colonies in lethally irradiated mice as a function of time (days) after an adenoviral vector encoding P1GF was administered.

**Figure 9** is a graph of the densitometric intensity of the gelatinolytic bands specific for matrix metalloprotein-9 (MMP-9) activation in cultured stem cells after administration of SDF-1, VEGF, or P1GF.

### DETAILED DESCRIPTION OF THE INVENTION

The present invention provides methods of isolating mammalian stem cells expressing the VEGF receptor VEGFR-1, as well as the therapeutical use thereof. These inventive methods involve binding a population of cells to a molecule that specifically binds to VEGFR-1 and isolating the cells that bind to the molecule. Preferably, the present inventive methods include further positively and/or negatively selecting for stem cells. These isolated mammalian stem cells expressing VEGFR-1, preferably further positively and/or negatively selected, can then be administered to a mammal to treat various conditions, which can involve inducing hematopoiesis, vasculogenesis and/or angiogenesis, myogenesis and neurogenesis to treat the condition.

The molecule that specifically binds VEGFR-1 of the present invention can be an antibody, a ligand, a peptide, a DNA, a small molecule, or any other suitable molecule. In order to be useful, the antibody, ligand, peptide, DNA, or small molecule must specifically bind to VEGFR-1.

In one embodiment of the present invention, the molecule that specifically binds VEGFR-1 is an antibody, which can be a monoclonal antibody, a functional fragment of an antibody, a chimerized antibody, a humanized antibody, or a fully human antibody. An antibody suitable in the context of the present invention specifically binds to the extracellular portion of the receptor. As used herein, unless otherwise indicated or clear from the context, antibody domains, regions and fragments are accorded standard definitions as are well known in the art. *See, e.g.,* Abbas et at, *Cellular and Molecular Immunology,* W.B. Saunders Company, Philadelphia, PA (1991).

The antibodies of the subject invention are preferably monoclonal. An especially preferred antibody of the present invention is termed clone 6.12, which is a mouse monoclonal antibody (MAb) that binds to soluble and cell surface-expressed VEGFR-1. A hybridoma cell line producing clone 6.12 has been deposited as ATCC number PTA-3344. Other antibodies of the present invention are produced by hybridomas that have been deposited and are publicly available. Such hybridomas include, but are not limited to, hybridomas KM1730 (deposited as FERM BP-5697), KM1731 (deposited as FERM BP-5718), KM1732 (deposited as FERM BP-5698), KM1748 (deposited as FERM BP-5699), KM1750 (deposited as FERM BP-5700) disclosed in International Application WO 98/22616 and in Australian accepted application no. AU 1998 50666 B2 and International WO 99/59636 and in Canadian application no. CA 2328893. In addition, bi-specific antibodies (BsAbs), which are antibodies that have two different antigen-binding specificities or sites, directed to KDR and VEGFR-1 are known. *See, e.g.,* U.S. Application No. 09/865,198 (Zhu); 60/301,299 (Zhu).

The molecule that specifically binds VEGFR-1 can also be a fragment of an antibody. Fragments of antibodies useful in the invention have the same binding characteristics as, or have binding characteristics comparable to, those of the whole antibody. Such fragments can contain one or both Fab fragments or the F(ab')₂ fragment. Such fragments can also contain single-chain fragment variable region antibodies, i.e., scFv, dibodies, or other antibody fragments. Preferably the antibody fragments contain all complementarity-determining regions of the whole antibody, although fragments containing fewer than all of such regions can also be functional.

If the antibody fragment is too short to be immunogenic, it can be conjugated to a carrier molecule. Some suitable carrier molecules include keyhole limpet hemocyanin and bovine serum albumin. Conjugation can be carried out by methods known in the art.

Antibodies of the present invention also include those for which binding characteristics have been improved by direct mutation, methods of affinity maturation, phage display, or chain shuffling. Affinity and specificity can be modified or improved by mutating CDRs and screening for antigen binding sites having the desired characteristics (see, e.g., Yang et al., J. Mol. Biol. 254: 392-403 (1995)). CDRs are mutated in a variety of ways. One way is to randomize individual residues or combinations of residues so that in a population of otherwise identical antigen binding sites, all twenty amino acids are found at particular positions. Alternatively, mutations are induced over a range of CDR residues by error prone PCR methods (see, e.g., Hawkins et al., J. Mol. Biol. 226: 889-896 (1992)). Phage display vectors containing heavy and light chain variable region genes are propagated in mutator strains of *E*. *coli* (see, e.g., Low et al, J. Mol. Biol. 250: 359-368 (1996)). These methods of mutagenesis are illustrative of the many methods known to one of skill in the art.

Specific antibody binding domains can be obtained from phage display libraries, wherein combinations of human heavy and light chain variable domains are displayed on the surface of filamentous phage (see, e.g., McCafferty et al., Nature, 348: 552-554 (1990); Aujame et aL, Human Antibodies, 8: 155-168 (1997)). Combinations of variable domains are typically displayed on filamentous phage in the form of Fabs or scFvs. The library is screened for phage bearing combinations of variable domains having desired antigen-binding characteristics. Preferred variable domain combinations display high affinity for a selected antigen and little cross-reactivity to other related antigens. By screening very large repertoires of antibody fragments, (see, e.g., Griffiths et al., EMBO J., 13, 3245-3260 (1994)), a good diversity of high affinity MAbs are isolated, with many expected to have sub-nanomolar affinities for the desired antigen.

The monoclonal antibody, a functional fragment of an antibody, a chimerized antibody, or a humanized antibody of the present invention can be prepared by using any of a number of techniques well known in the art. These methods include, but are not limited to, the hybridoma technique described by Kohler and Milstein (Nature, 256:495-497(1975)), the human B cell hybridoma technique (Kozbor et al., *Immunology Today,* 4:72(1983)), the EBV-hybridoma technique (Cole et al., Monoclonal Antibodies and Cancer Therapy, Alan R. Liss, Inc., 77-96 (1985)), and the trioma techniques. For an overview of antibody production methods, see Hartlow, E. et al., Antibodies: A Laboratory Manual, Cold Spring Harbor Laboratory Press, Cold Spring Harbor, N.Y., 1988.

Fragments of antibodies can be produced by cleaving a whole antibody or by expressing DNA that encodes the fragment. Fragments of antibodies can also be prepared by methods described in Lamoyi et al., J. Immunol. Methods, 56: 235-243 (1983) and by Parham, J. Immunol. 131: 2895-2902 (1983).

Antibody molecules or fragments can be purified by known techniques, e.g., immunoabsorption or immunoaffinity chromatography, chromatographic methods such as HPLC (high performance liquid chromatography), or a combination thereof, etc.

Cloning DNA encoding the molecule into a vector of an appropriate expression system can also produce the molecule that specifically binds VEGFR-1. For example, HCMV vectors designed to express either human light chains of human heavy chains in mammalian cells can be utilized to express antibodies of the present invention. *See, e.g.,* U.S. Patent No. 5,840,299; Maeda, et al., Hum. Antibod Hybridomas, 2: 124-134 (1991). Such vectors can contain a promoter and enhancer for high level transcription of the constructs, e.g., the human cytomegalovirus (CMV), replication origins and selectable markers functional in mammalian cells and *E*. *coli.*

Preferred host cells for transformation of vectors and expression of molecule that specifically bind VEGFR-1 of the present invention are mammalian cells, e.g., COS-7 cells, chinese hamster ovary (CHO) cells, and cell lines of lymphoid origin such as lymphoma, myeloma, or hybridoma cells. Other eukaryotic host, such as yeasts, can be alternatively used.

Also alternatively, the DNA encoding the molecule that specifically binds VEGFR-1 can be cloned into vectors derived from viruses such as adenovirus, adeno-associated virus, herpesvirus, retrovirus or lentivirus. Gene expression is controlled by inducible or uninducible regulatory sequences. A more detailed description of vector cloning is described subsequently.

In embodiments wherein the molecule that specifically binds VEGFR-1 is a peptide or DNA, the molecule that specifically binds VEGFR- 1 can alternatively be prepared using standard solid phase (or solution phase) peptide synthesis methods, as is known in the art. In addition, the DNA can be synthesized using commercially available oligonucleotide synthesis instrumentation and produced recombinantly using standard recombinant production systems. Production using solid phase peptide synthesis is necessitated if non-gene-encoded peptides are to be included. Provided that the peptide or DNA specifically binds VEGFR-1, any suitable peptide or DNA can be used in the context of the present invention.

Alternatively, the molecule that specifically binds VEGFR-1 can be a small molecule. Small molecules include, for example, lipids and polymers of polysaccharides, as well as derivatives thereof, such as, e.g., lipopolysaccharides. Again, any suitable small molecule that binds VEGFR-1 can be used in the context of the present invention.

In one aspect of the invention, the molecule that specifically binds VEGFR-1 can be fused to additional amino acid residues such as a peptide tag to facilitate isolation or purification.

Either before or after the stem cells are isolated using a molecule that specifically binds to VEGFR-1, the stem cells can be further enriched, i.e. purified by additional rounds of isolation using the methods described above for VEGFR-1 stem cells. For example, the stem cells can be isolated by positive selection for one or more markers characteristic of stem cells (positive selection markers). Such markers include, for example, CD34, and AC133. The stem cells can also be further isolated by negative selection for one or more markers characteristic of mature cells, for example, CD38 (negative selection markers).

Accordingly, in a further embodiment the methods additionally comprise binding the mammalian cells to one or more positive selection markers and isolating the cells that are bound to the marker from cells that are unbound to the marker.

In one example of a positive selection marker, stem cells can also be further isolated with the AC133 antibodies described by Yin et al. in Blood 90, 5002-5112 (1997) and by Miraglia et al. in Blood 90, 5013-5021 (1997). The AC133 antigen is expressed on stem cells, but not on mature cells. The AC133 antibodies can be prepared in accordance with Yin et al., *supra,* or purchased from Miltenyi Biotec.

It should be appreciated that these methods can also be used to remove cells that do not contain markers that specifically bind stem cells, that is, to remove cells expressing markers characteristic of mature cells by negative selection. Markers characteristic of mature cells include CD1, CD2, CD3, CD4, CD5, CD8, CD10, CD11b, CD13, CD14, CD15, CD16, CD19, CD20, CD24, D25, CD28, CD29, CD33, CD36, CD38, CD41, CD41 a, CD56, CD66b, CD66e, CD69, and glycophorin A. As discussed previously, cells lacking some or all of these markers are referred to as Lin-.

Therefore, the preferred methods of the present invention optionally additionally comprise binding the mammalian stem cells to one or more negative selection markers and isolating the cells that are not bound to the marker from cells that are bound to the marker. In this case, the cells that are not bound to the negative selection marker contain stem cells and are of interest. Negative selection can be used either before or after positive selection. Stem cells can be purified/enriched by means of positive selection, or by a mixture of both positive and negative selection, which can be performed in any order.

The method of isolation/purification based on the presence or absence of additional marker(s) is not limited to the use of one marker, i.e. methods using one or more additional positive and/or negative selection markers can be iterated as many times as needed so as to enrich for the stem cell population.

The positive and negative selection markers can be an antibody, a ligand, a peptide, a DNA, a small molecule, or any other suitable molecule (as described previously). Preferably, the positive selection marker specifically binds to AC133, CD34, and anti-angiopoietin-1 (Tie-2) and more preferably the negative selection marker specifically binds Lin and CD38. Methods for producing such markers are known in the art and many are commercially available. *See, e.g.,* U.S. Patent No. 5,130,144; U.S. Patent No. 5,061,620; U.S. Patent No. 5,035,994. Accordingly, the preferred cells of the present invention specifically bind VEGFR-1, CD34, AC133, and specifically do not bind Lin and CD38 (VEGFR-1+ CD34+ AC133+ Lin- CD38-).

In the context of the present invention, any suitable cell can be isolated, provided that the cell expresses VEGFR-1. Preferably, the cell is a human stem cell. Stem cell is a term used herein to describe a cell that can give rise to one or more tissue types. Totipotent stem cells are cells that can give rise to a fully functional organism as well as to every cell type of the body. Pluripotent stem cells are capable of giving rise to virtually any tissue types, but not to a functioning organism. Multipotent stem cells are more differentiated cells (that is, their possible lineages are less plastic and more determined) and thus can give rise only to a limited number of tissues. A stem cell can also be bipotent, monopotent, or a progenitor cell.

The mammalian stem cells of the present invention encompass all these different types of cells, regardless of whether the cells are totipotent, pluripotent, multipotent, bipotent, monopotent, etc.

There are many potential sources for the population of cells of the present invention. Embryonic non human stem cells are derived from the inner cell mass of a blastocyst (a very early embryo). Embryonic non human germ cells are collected from fetal tissue at a somewhat later stage of development (from a region called the gonadal ridge). Adult stem cells are derived from mature tissue. Examples of adult stem cells include liver cells that proliferate following partial hepatectomy (hepatic cells), hematopoietic cells that can reconstitute the blood following lethal irradiation or chemotherapy, satellite cells that repair damaged skeletal muscle, keratinocyte precursors that participate in wound healing, and neural cells involved in brain repair. In addition to repairing damage, stem cells play a key role in ongoing tissue homeostasis, for example in maintaining the blood and skin throughout life.

Preferably, the source population of cells from which isolated mammalian stem cells are derived can be any natural or non-natural mixture of cells. The population of cells can thus be from an embryonic non human mammal, or from the post-natal mammal, including fetal liver, umbilical cord blood, a yolk sac of a mammal, a mature spinal cord, bone marrow, or an adult peripheral blood sample. The cells can also be from the central nervous system, including the meninges.

Preferably, the isolated stem cells of the present invention are hematopoietic stem cells (HSCs), although the isolated stem cells of the present invention can also be endothelial stem cells, muscle stem cells, and neural stem cells. The muscle cells can be, for example, skeletal muscle cells, cardiac muscle cells, and smooth muscle cells, which include, for example, the muscle cells of blood vessels and of the gastrointestinal tract.

A suitable source of the population of cells can be harvested from a mammalian donor by methods known in the art. For example, the cells can be harvested from the hematopoietic microenvironment. In addition, circulating peripheral blood, preferably mobilized (i.e., recruited), can be removed from a patient. Alternatively, bone marrow can be obtained from a mammal, such as a human patient, undergoing an autologous transplant.

The population of cells obtained is then contacted with or exposed to the molecule that specifically binds VEGFR-1 or the positive and/or negative selection marker. The cells that express VEGFR or the marker bind to the molecule, permitting separation and isolation of the cells of interest. If the cells do not internalize the molecule or marker, it can be separated from the cell by methods known in the art. For example, antibodies can be separated from cells by a short exposure to a solution having a low pH, or with a protease such as chymotrypsin. It should be appreciated that in the preferred embodiment wherein the isolated cells expressing VEGFR-1 are positively and/or negatively enriched for stem cells, one of skill would recognize that isolation of the cells expressing VEGFR-1 can be accomplished prior to, concurrently with, or subsequent to positive and/or negative selection of the cells.

The molecule or marker used for isolating the populations of mammalian stem cells of interest is advantageously conjugated with labels that expedite identification and separation. Examples of such labels include magnetic beads; biotin, which can be identified or separated by means of its affinity to avidin or streptavidin; fluorochromes, which can be identified and/or separated by means of a fluorescence-activated cell sorter (FACS), and the like.

Any technique can be used for isolation as long as the technique does not unduly harm the cells. Many such methods are known in the art. In a preferred embodiment, a labeled binding molecule or marker is bound to the desired cells, and a mechanical cell sorter that detects the presence of the label separates the labeled cells. The preferred mechanical cell sorter is a FACS machine, which are commercially available. *See generally* Orfao & Ruiz-Arguelles, Clin. Biochem., 29(1): 5-9 (1996).

In alternative embodiments, the cells can be isolated using magnetic separation with magnetic beads or attachment to a solid support, for example, nitrocellulose, agarose beads, polystyrene beads, hollow fiber membranes, magnetic beads, and plastic petri dishes. The exact conditions and duration of incubation for these methods will depend upon several factors specific to the system employed, as is well known in the art.

In a particularly preferred variation of the method described above, blood is withdrawn directly from the circulating peripheral blood of a donor. The blood is percolated continuously through a column containing the solid phase-linked molecule or marker, such as an antibody to VEGFR-1, to capture the desired cells, including hematopoietic stem cells, endothelial stem cells, muscle stem cells and neural stem cells. The cell-depleted blood is returned immediately to the donor's circulatory system by methods known in the art, such as hemaphoresis. The blood is processed in this way until a sufficient number of stem cells binds to the column. The desired cells are then isolated from the column by methods known in the art. This method allows rare peripheral blood stem cells to be harvested from a very large volume of blood, sparing the donor the expense and pain of harvesting bone marrow and the associated risks of anesthesia, analgesia, blood transfusion, and infection.

Alternatively, the molecule that specifically binds VEGFR-1 or positive or negative selection marker can be linked to magnetic colloids for capture of unwanted cells on a column surrounded by a magnetic field. This system is currently available through StemCell Technologies Inc., Vancouver, British Columbia; Canada. The remaining cells that flow through the column for collection are enriched in cells that do not express the cell surface proteins that the molecule is directed against.

In another aspect of the present invention, a ligand that specifically binds to VEGFR-1, which is preferably PIGF, is provided. Any ligand can be used in the context of the present invention, such as a peptide, an antibody, a DNA, a small molecule, or any other suitable molecule, provided that the ligand activates VEGFR-1. Methods of selection, as well as production, of a suitable protein, antibody, DNA, and small molecule have been described previously.

Mutants of the ligand can be also be used in the present invention. It should be appreciated that a skilled artisan can easily create mutants according to techniques known in the art.

Activation, in the context of the present invention, means that VEGFR-1 signaling is increased. As VEGFR-1 functions via dimerization and transduction of an intracellular signal through tyrosine phosphorylation, methods of determining receptor phosphorylation, which are well known in the art and include, for example, measurement of phosphotyrosine with monoclonal antibodies or radioactive labels, can be used to determine activation.

The present invention also encompasses the use of compositions comprising stem cells expressing VEGFR-1. Preferably, the used compositions comprise stem cells expressing VEGFR-1 and more preferably, stem cells that have been enriched by positive and/or negative selection. In another preferred embodiment, the compositions comprise the VEGFR-1 ligand P1GF. In one aspect, the ligand specific for VEGFR-1 is present as a vector containing the gene that encodes the ligand.

Any vector appropriate for transferring an exogenous gene to a cell is included within the scope of the present methods. Preferably, the vector is a viral vector. Examples of viral vectors employed in accordance with the present inventive method include, but are not limited to, retroviral vectors, adenoviral vectors, adeno-associated viral vectors, herpesviral vectors, SV40 viral vectors, polyoma virus vectors, papilloma virus vectors, picnoravirus vectors, vaccinia virus vectors, or other suitable vectors. Preferably, the viral vector is an adenoviral vector.

Such adenoviral vectors can be modified human adenoviruses such as Ad2 or Ad5, wherein genetic elements necessary for the virus to replicate *in vivo* have been removed; e.g. the E1 region, which adenoviral vectors are generally referred to as replication incompetent. An expression cassette containing the gene encoding the ligand that specifically binds VEGFR-1 can then be inserted into the adenoviral genome.

The skilled artisan will be able to incorporate an expression cassette into the nucleic acid sequence of the vector. Methods for incorporating expression cassettes into viral vectors are well known in the art (see e.g., Sambrook, et al Molecular Cloning: a Laboratory Manual, 2d edition, Cold Spring Harbor Press (1989)) and include direct cloning, site specific recombination using recombinases, such as the flp recombinase or the cre-lox recombinase system (reviewed in Kilby et al. Trends in Genetics, 9: 413-21 (1993)), homologous recombination, and other suitable methods of constructing a recombinant vector.

In addition to viral vectors, any non-viral vector capable of expression upon infection of target cells can be used in the present methods, for example, a plasmid.

It is understood that the compositions, where used in a mammal for the purpose of prophylaxis or treatment, will be administered in the form of a composition additionally comprising a pharmaceutically acceptable carrier.

Suitable pharmaceutically acceptable carriers include, for example, one or more of water, saline, phosphate buffered saline, dextrose, glycerol, ethanol and the like, as well as combinations thereof. Pharmaceutically acceptable carriers can further comprise minor amounts of auxiliary substances such as wetting or emulsifying agents, preservatives or buffers, which, enhance the shelf life or effectiveness of the binding proteins. The compositions of the injection can, as is well known in the art, be formulated so as to provide quick, sustained or delayed release of the active ingredient after administration to the mammal.

The compositions can be in a variety of forms. These include, for example, solid, semi-solid and liquid dosage forms, such as tablets, pills, powders, liquid solutions, dispersions or suspensions, liposomes, suppositories, injectable and infusible solutions. The preferred form depends on the intended mode of administration and therapeutic application.

Such compositions are prepared in a manner well known in the pharmaceutical art. In making the composition the active ingredient will usually be mixed with a carrier, or diluted by a carrier, and/or enclosed within a carrier which can, for example, be in the form of a capsule, sachet, paper or other container. When the carrier serves as a diluent, it can be a solid, semi-solid, or liquid material, which acts as a vehicle, excipient or medium for the active ingredient. Thus, the composition can be in the form of tablets, lozenges, sachets, cachets, elixirs, suspensions, aerosols (as a solid or in a liquid medium), ointments containing for example up to 10% by weight of the active compound, soft and hard gelatin capsules, suppositories, injection solutions, suspensions, sterile packaged powders and as a topical patch.

The present compositions can be administered to a mammal for prophylactic and/or therapeutic treatments of various conditions. Treatment of the conditions can involve induction of hematopoiesis, vasculogenesis and/or angiogenesis, myogenesis and/or neurogenesis. Moreover, treatment can involve mobilization of stem cells to the area of administration, as well as stimulation of the stem cells to proliferation and/or differentiation.

The identification of those mammals that would benefit from administration of the present compositions is well within the ability and knowledge of one skilled in the art. A clinician skilled in the art can readily determine, for example, by the use of clinical tests, physical examination and medical/family history, if an individual is suffering from any suitable condition.

There are numerous conditions that can be treated using the present inventive methods. For example, the mammal can have a genetic disease such as sickle cell anemia or thalassemia or a disorder which requires receipt preferably of an autologous transplant of tissue or organ(s) or receipt of a homologous or heterologous transplant of tissue or organ(s). The mammal can also have undergone or is receiving chemotherapy or radiation therapy, all of which conditions deplete or damage the mammal's blood. Additionally, the mammal in need of hematopoiesis can have suffered a hemorrhage or have incurred a wound that requires healing. The wound can be an acute wound, such as those caused by burns and contact with hard and/or sharp objects, including recovery from surgery, such as cardiovascular surgery, cardiovascular angioplasty, carotid angioplasty, and coronary angioplasty. The wound can also be a chronic wound, examples of which include ulcers, such as vascular ulcers and diabetic ulcers.

Inducing vascularization is especially effective in increasing cardiac or peripheral (i.e. limb) vascularization. Therefore, the method is especially effective in treating cardiac and peripheral ischemia.

The stem cells also can be recruited into a site that requires new cells and tissues. For example, hematopoietic stem cells can be mobilized (i.e., recruited) into the circulating peripheral blood by means of cytokines, such as, for example, G-CSF, GM-CSF, VEGF, SCF (c-kit ligand) and bFGF, chemokines, such as SDF- 1, or interleukins, such as interleukins 1 and 8. Stem cells can also be recruited to the circulating peripheral blood of a mammal if the mammal sustains, or is caused to sustain, an injury.

Mobilization of a large number of stem cells to the peripheral circulation also facilitates isolation of large numbers of stem cells expressing VEGFR-1, which can be accomplished using the methods described herein. These isolated stem cells can then be used for therapeutic treatments, which are discussed herein, as well as bone marrow transplantation and reconstitution. In addition, these stem cells can directly or intravenously be used for restoring function to the ischemic myocardium, reconstitute pancreatic islet cells, or regenerate damaged neurons.

It should be appreciated that the compositions used according to the present invention can be administered to any mammal. Specifically, the compositions can be administered to a human. Furthermore, in embodiments wherein the composition comprises cells expressing VEGFR-1, the cells can be autologous, homologous, or heterologous to the mammal to which the cells are administered. Preferably, however, the cells are autologous to the mammal to which the cells are administered.

In another aspect of the invention, the compositions can be chemically or biosynthetically linked to one or more therapeutic agents. The compositions can also be administered in combination with any other method of treatment of the various known in the art, examples of which are conventionally known in the art.

The invention further contemplates compositions to which target or reporter moieties are linked, thereby specifically targeting the compositions to the area of treatment in the mammal.

In therapeutic applications, compositions are administered to a patient already suffering from the condition, in an amount sufficient to cure or at least partially arrest the condition. An amount adequate to accomplish this is defined as a "therapeutically effective dose." Amounts effective for this use will depend upon the severity of the condition and the general state of the mammal's own immune system. Dosing schedules will also vary with the disease state and status of the mammal, and will typically range from a single bolus dosage or continuous infusion to multiple administrations per day (e.g., every 4-6 hours), or as indicated by one of skill and the mammal's condition.

In prophylactic applications, compositions containing the present compositions are administered to a mammal not presently suffering from the condition, in an amount sufficient to prevent at least partially the effects of the condition. Such an amount is also defined to be a "therapeutically effective dose." In this use, again the precise amounts again depend upon the mammal's state of health and general level of immunity, as well as dosing schedules, which are described previously.

For the purpose of this invention, the composition can also be administered by various routes, for example by oral, intravenous, intraperitoneal, subcutaneous, intracerebrospinal, subcutaneous, intrathecal, intramuscular, inhalation, or topical administration.

Growth and differentiation factors, the microenvironment, including contact with neighboring cells, the extracellular matrix, and the local milieu all play a role in determining cell phenotype and function. *See, e.g.,* Hay, Collagen and other matrix glycoproteins in embyogenesis. In Cell Biology of Extracellular Matrix, E.D. Hay, ed. (New York: Plenum Press), 419-462 (1991); Studer et al., J. Neurosci., 20: 7377-83 (2000). Specifically, growth and differentiation factors are molecules that stimulate cells to proliferate and/or promote differentiation of cell types into functionally mature forms.

A person of ordinary skill would recognize that, in some embodiments of the invention, growth and differentiation factors can be administered in combination with the compositions for use according to the present invention. For example, growth and differentiation factors can be administered in combination with cells expressing VEGFR-1 in order to direct the administered cells to proliferate and differentiate in a specific manner. In alternative embodiments, growth and differentiation factors can be administered in combination with the ligand, e.g., PIGF, that specifically binds VEGFR-1, thus recruiting cells to the site of administration. One of ordinary skill would recognize that growth and differentiation factors can be administered prior to, concurrently with, or subsequent to the administration. In addition, administration of the growth and/or differentiation factors can be repeated. In a further embodiment, growth and or differentiation factors can be provided to the cells expressing VEGFR-1 while the cells are maintained in culture either prior to, concurrently with, or subsequent to the isolation methods of the present invention.

It is envisioned that a growth and/or differentiation factor can be, for example, SCF (c-kit ligand), Flk-2/Flt-3 ligand, or thrombopoietin alone or in combination with P1GF, or VEGF, which promote proliferation and expansion of stem cells.

In another aspect of the invention, the isolated stem cells of the present invention can comprise a DNA of interest into a mammal.

The DNA of interest can be introduced into the isolated cells by methods known in the art and described in, for example, Mulligan, et al., U.S. Patent No. 5,674,722. Alternatively, the DNA can be cloned into vectors derived from viruses such as adenovirus, adeno-associated virus, herpesvirus, retrovirus or lentivirus. Gene expression can be controlled by inducible or uninducible regulatory sequences.

Generally, a DNA of interest is a gene that corrects or compensates for an underlying protein deficit or, alternately, that is capable of down-regulating a particular gene, or counteracting the negative effects of its encoded product, in a given condition. Moreover, a therapeutic gene can be a gene that mediates cell killing, for instance, in the therapy of cancer. Alternatively, the DNA of interest can encode a protein of interest.

Some examples of proteins of interest in the context of the present invention include PIGF, VEGF, Factor VIII, von Willebrand factor, insulin, tissue plasminogen activator, any of the interleukins, or any other growth or differentiation factor, specific examples of which have been described previously. Preferably, the protein of interest is PIGF, which is encoded by the DNA.

It is to be understood and expected that variations in the principles of invention herein disclosed can be made by one skilled in the art and it is intended that such modifications are to be included within the scope of the present invention.

The examples that follow further illustrate the invention, but should not be construed to limit the scope of the invention in any way. Detailed descriptions of conventional methods, such as those employed in the construction of vectors and plasmids, the insertion of genes encoding polypeptides into such vectors and plasmids, the introduction of plasmids into host cells, and the expression and determination thereof of genes and gene products can be obtained from numerous publication, including Sambrook, J. et al., (1989) Molecular Cloning: A Laboratory Manual, 2nd ed., Cold Spring Harbor Laboratory Press. *See also* Rafii et al., Nature Med., Vol. 8, No. 8 (August 2002).

### EXAMPLES

### Anti-VEGFR-1 MAb Clone 6.12

Clone 6.12 is a mouse monoclonal antibody (MAb) that binds to soluble and cell surface-expressed VEGFR-1. It was produced using standard techniques from ScFv 6.12, which comprises the V_{L} and V_{H} domains of MAb 6.12. A hybridoma cell line producing clone 6.12 has been deposited as ATCC number PTA-3344.

### Anti-VEGFR-1 MAb MF-1

The rat anti-VEGFR-1 monoclonal antibody was developed through a standard hybridoma technique. Eight weeks old rats were primed intraperitoneally (i.p.) with 100 µg of VEGFR-1 Fc (constant region) recombinant protein (R&D Systems, Minneapolis, MN) mixed with complete Freunds adjuvant. Then, the rats were boosted three times prior to fusion with the same protein mixed with incomplete Freunds adjuvant.

Hybridoma cells were generated by fusing myeloma cells P3x63Ag8.653 with spleen cells and bone marrow cells from immunized rats. Anti-VEGFR 1 specific clones were selected using VEGFR-1 alkaline phosphatase (AP) recombinant protein in ELISA-based binding and blocking assays. Positive clones were subcloned by limiting dilution.

Anti-VEGFR-1 MAbs from hybridomas were obtained via continuous feed fermentation in serum-free medium. The MAbs were purified from serum-free conditioned media by affinity chromatography using Gamma-bind protein G-Sepharose. The MAbs used in *in vivo* studies were tested for endotoxin using the Pyrogent plus® Limulus Amebocyte Lysate kit (BioWhittaker, Walkersville, MD). All antibody preparations used in animal studies contained ≤1.25 EU/ml of endotoxin. Anti-VEGFR-1 polyclonal antibodies were generated from recombinant VEGFR-1 AP protein immunized rabbit and purified by Gamma-bind protein G column (Amersham Pharmacia Biotech, Uppsala, Sweden).

The immunochemical properties of anti-VEGFR-1 MAbs were characterized in ELISA-based binding and blocking assays as well as BIAcore analysis for affinity. Binding assays were performed by coating 96-well microtiter plates (Falcon Flexible plate, Becton Dickinson, Bedford, MA) with 50 ng/well VEGFR AP or VEGFR-2 AP protein overnight at 4° C. Wells were blocked by adding 200 µl of phosphate-buffered saline containing 5% bovine serum, 0.05% Tween 20 (blocking buffer) and incubating for 2 hrs at room temperature (RT). Wells were then washed (5x) and incubated for 1 hr at RT with various concentrations of MAbs at 50 µl diluted in blocking buffer. Wells were again washed (5x) and incubated with 50 µl of goat anti-rat IgG-HRP (BioSource International, Camarillo, CA) for 1 hr at RT. Wells were washed (5x) for a final time and then incubated with 50 µl of 3,3', 5,5'-tetra-methylbenzidine (TMB) substrate (Kirkegaard and Perry Lab Inc., Gaithersburg, MD) for 15 mins at RT. The reaction was stopped by adding 50 µl of 1 M Phosphoric Acid (H₃PO₄) and wells read at 450 nm on a microtiter plate reader.

For VEGFR-1/VEGF or P1GF blocking assays, wells were coated with 100 ng of VEGF or P1GF (R & D Systems, Minneapolis, MN) overnight at 4°C. Wells are blocked as described above and then incubated for 1 hr at RT with 100 ng of VEGFR-1 AP that had been preincubated for 1 hr with various concentrations of MAb. Wells were washed and incubated with p-nitrophenyl phosphate (PNPP, Sigma, St. Louis, MO). Color was developed for 30 mins at RT and was then read at 405 nm on a microtiter plate reader.

The binding kinetics of anti-VEGFR-1 MAbs to VEGFR was determined using BIAcore biosensor (Pharmacia Biosensor). VEGFR Fc fusion protein was immobilized onto a sensor chip and the MAbs was injected at concentrations ranging from 3.125 nM to 50 nM. Sensorgrams were obtained at each concentration and were evaluated using the program, BIA Evaluation 2.0, to determine the ratio of rate constant kon/koff for Kd value.

### Adenoviral Vectors

AdPIGF is an Adenovirus type 5 (Ad5) -derived E1a- E3-deficient (E1a-E3-E4+) adenoviral vector with an expression cassette in the E1a region containing the human P1GF cDNA driven by the cytomegalovirus (CMV) major immediate/early promoter/enhancer.

The control vector, AdNull, is an identical adenoviral vector without a transgene in the expression cassette.

### Example 1

The present example investigates expression of VEGFR-1 on stem cells. Specifically, neutralizing and non-neutralizing MAbs were generated that selectively bind either human or mouse VEGFR-1 using standard techniques. Human fetal liver (FL) (15-16 weeks of gestation) and cord blood (CB) were obtained from fetuses. CD34⁺ cells were isolated from FL and CB using standard immunomagnetic techniques (MACS; Miltenyi Biotech). Flow cytometry analysis indicated 85-95% purity of the CD34⁺ fraction with 45-55% recovery. CD34⁺ cells (1 × 10⁵ FL or CB) were incubated for 30 minutes at 4° C with fluorescein isothiocyanate (FITC) or phycoerythrin (PE) conjugated MAbs; human CD34-PE, CD38-PE (Beckton Dickinson), CD15-PE (Immunotech), AC133-PE (Miltenyi Biotech), CD14-PE (PharMingen), VEGFR-1-FITC (clone 6.12; ImClone Systems). The cells were analyzed by two-color flow cytometry using a Coulter Elite flow cytometer.

Using FTTC labeled MAbs to the extracellular domain of VEGFR-1, we found that VEGFR-1 is expressed on 6.0±0.5% (N=4) and 4.3±0.3% (N=6) of human FL and CB derived CD34⁺ and AC133⁺ cells. Approximately 85% of the VEGFR positive cells were CD15 and CD14 negative, indicating that these cells are not mature CD15 or CD14 myeloid cells.

### Example 2

This example investigates the stem cell potential of VEGFR-1⁺ cells. Specifically, the stem cell potential of VEGFR-1⁺ cells was evaluated in *in vivo* repopulating assays including non-obese diabetic (NOD) - severe combined immunodeficiency (SCID) mouse repopulating cells.

To this end, freshly isolated human CB CD34⁺, purified CD34⁺VEGFR-1⁺ and CD34⁺VEGFR-1- cells were transplanted into sublethally (3.5 Gy) irradiated NOD/SCID mice. Briefly, Human CD34⁺ mononuclear cells (MCs) were isolated from CB and incubated with biotinylated anti-VEGFR-1 Ab (ImClone Systems). CD34⁺V-EGFR-1⁺ MCs were separated using immuno magnetic separation (Miltenyi Biotech) according to the manufacturer's instructions. The purity of the VEGFR-1⁺ MCs preparations was assessed by flow cytometry using the fluorescein isothiocyanate (FITC)-conjugated anti-VEGFR-1 antibody (clone 6.12; ImClone Systems) and was found to be 85-95%. The immunocompromised NOD/SCID mice (Jackson laboratory) were handled under bio-clean conditions maintained in microisolators. Transplant recipients (aged 8 weeks) were treated with an irradiation dose of 3.5 Gy administered from from a ¹³⁷Cs γ-ray source at a dose rate of approximately 0.90 Gy/min. Transplantation of 4 × 10⁴ to 2 ×10⁵ human CD34⁺VEGFR- 1⁺ cells in 0.3 ml volume of Iscove's modified Dulbecco's medium (IMDM) (Sigma) by tail vein injections followed within 6 hours of irradiation. The mice were sacrificed at 6 to 8 weeks after transplantation and bone marrow mononuclear cells (BMMCs) were collected from femurs.

Six weeks after transplantation the number of human derived CD45⁺ hematopoietic cells was quantified in NOD/SCID mouse BM by flow cytometer. To determine the percentage of human MCs, each sample (10⁵ MCs) was stained with antihuman CD45-FITC and anti-murine CD45-PE (PharMingen). The cells were then analyzed by two-color flow cytometry using a Coulter Elite flow cytometer.

Transplantation of as few as 10⁴ CD34⁺VEGFR-1⁺ cells was sufficient to support engraftment of human NOD/SCID-repopulating cells. These data suggest that CD34⁺VEGFR-1⁺ cells contain a population of cells with stem cell potential.

### Example 3

The present example investigates expression of VEGFR-1 in murine stem cells. To assess whether VEGFR-1 is also expressed in the murine stem cells, a cohort of mice were treated with 5-fluorouracil (5FU), allowing for enrichment of non-cycling hematopeotic stem cells (HSCs). Briefly, mice were treated with a sublethal dose of 5FU (300 mg/kg), resulting in apoptosis of rapidly cycling progenitors and precursors, while non-cycling quiescent cells mostly of stem cell potential are spared. This is followed by rapid reactivation of Go stem cells and reconstitution of lymphohematopoietic cells. The cells were then analyzed by two-color flow cytometry using a Coulter Elite flow cytometer.

Flow cytometric analysis showed that 5.0 ± 0.3% (N=6) of 5FU-pretreated BALB/c BMMCs expressed VEGFR-1⁺. Within the VEGFR-1⁺ population, 35.7±0.7% were Sca-1⁺ while 31.4±0.3% were c-kit positive.

### Example 4

This example investigates treatment of a condition associated with a reduction in stem cells, specifically, destruction of the bone marrow. To determine the BM-repopulating capacity of VEGFR-1⁺ BALB/c mouse BMMCs, varying cell doses of purified population of VEGFR-1⁺ cells obtained from 5FU-pretreated BALB/c BMMCs (as described previously) were transplanted into lethally irradiated (9 Gy) syngeneic mice.

Fifteen BALB/c donor mice were injected intravenously with 5FU (150 mg/kg) 2 days prior to marrow cell collection. In all experiment, donor mice were killed by cervical dislocation and the femurs and tibias were removed under aseptic conditions. BMMCs were obtained by flushing both femoral and tibial bones with 3 ml of cold IMDM (GIBCO-BRL Life Technologies) containing 20% fetal calf serun (FCS). Murine VEGFR-1⁺ BMMCs were separated using immuno-magnetic separation (Miltenyi Biotech) according to the manufacturer's instructions. The purity of the VEGFR-1⁺ BMMCs preparations was assessed by flow cytometry using the fluorescein isothiocyanate (FITC)-conjugated anti-VEGFR-1 antibody (clone MF-1; ImClone Systems) and was found to be 88-95%. Separated VEGFR-1⁺ and VEGFR-1- cells (10⁴) were incubated for 30 minutes at 4° C with FITC or PE conjugated MAbs; Sca-1(Ly6A/E)-PE, murine c-kit-PE, CD34-FITC, CD11b-PE, CD45-PE (PharMingen). The cells were analyzed by two-color flow cytometry using a Coulter Elite FCM.

The recipient BALB/c mice (8 mice in each group) were lethally irradiated (9 Gy) and intravenously (i.v.) injected with serial cell doses (10⁵, 10³, 10² and 10) VEGFR-1⁺ or VEGFR-1- BMMCs after irradiation on day 0. Survival was monitored every day beyond day 150.

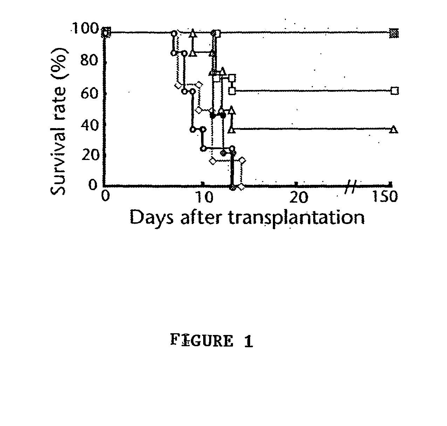

Results are shown in Figure 1, which is a graph of the survival (%) of the mice as a function of time (days). All the mice transplanted with various doses of VEGFR-1- cells died within 14 days, whereas 38%, 63%, and 100% of mice transplanted with 10²,10³ and 10⁵ VEGFR-1⁺ BMMCs survived beyond 150 days, respectively (Fig. 1). Transplantation of even as many as 10⁵ purified BMMCs VEGFR-2⁺ (Flk-1) cells obtained from 5FU-pretreated BALB/c BMMCs into lethally irradiated syngeneic failed to rescue the lethally irradiated mice. Remarkably, 42% and 75% of mice transplanted with 10³ and 10⁵ VEGFR-2- (Negative) BMMCs survived beyond 150 days.

### Example 5

This example investigates treatment of a condition associated with stem cells, specifically, destruction of the bone marrow. To assess the long-term BM-repopulating capacity of the VEGFR-1⁺ cells, BMMCs VEGFR-1⁺Sca-1⁺ cells obtained from 5FU-pretreated C57BL/6-Ly5.2. BMMCs were transplanted into lethally irradiated C57BL/6-Ly5.1 mice (N=6).

Long-term reconstitutive ability was assessed using purified VEGFR-1⁺ and/or Sca-1⁺ 5FU-treated BM cells in the syngeneic BM cell transplantation system. After collecting the BMMCs from 5FU-treated C57BL/6Ly5.2 mice 2 days after, VEGFR-1^{+/-} and Sca-1^{+/-} BMMCs were separated by using MoFlo flow cytometer/cell sorter. VEGFR-1⁺ or VEGFR-1- BMMCs (10³) were transferred into lethally irradiated (9 Gy) C57BL/6Ly5.1 mice (8 mice in each dose group). Four months after the transplantation, peripheral blood mononuclear cells (PBMCs) were collected from retro-orbital plexus and stained with FITC and PE conjugated MAbs; Ly5.1-PE, Ly5.2-FITC, murine CD11b-PE, B220-PE, Gr-1-PE, Thy-1-PE (PharMingen). The cells were analyzed by two-color flow cytometry using a Coulter Elite flow cytometer.

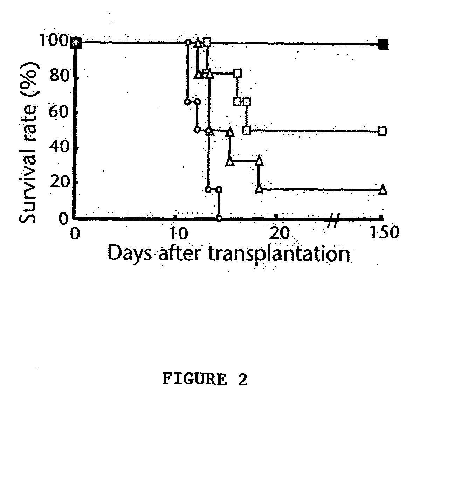

Transplantation of as low as 10³ VEGFR-1⁺Sca-1⁺ and VEGFR-1⁺Sca-1-BMMCs rescued lethally irradiated mice. Results are shown in Figure 2, which is a graph of the survival (%) of the mice as a function of time (days). Further, four months after transplantation, approximately 85% of myeloid and lymphoid lineage cells in peripheral blood expressed were of donor (Ly5.2) origin.

### Example 6

The present example investigates the role of VEGFR-1 and VEGFR-2 in the production of stem cells. To examine the functional role of VEGFR-1 and VEGFR-2 expression in the reconstitution of hematopoiesis, a model where the BM was suppressed with sublethal dose of 5FU (300 mg/kg) was utilized. In this model, treatment of mice with 5FU results in apoptosis of rapidly cycling progenitors and precursors, while non-cycling quiescent cells mostly of stem cell potential are spared. This is followed by rapid reactivation of Go stem cells resulting in reconstitution of lymphohematopoietic within 4 weeks.

To assess the significance of VEGFR-1 and VEGFGR-2 signaling in mediating reconstitution of hematopoeisis during 5FU-induced marrow suppression and recovery, cohorts of BALB/c mice were treated with neutralizing MAb to either VEGFR-1 or VEGFR-2 after 5FU treatment. MAb were prepared using standard techniques. Initially in 2 to 3 day intervals and later on a weekly basis, retro-orbital blood was collected with capillary pipettes (Unopette, Fisher Scientific). Total white blood cells were then counted using a Neubauer hematocytometer (Fisher Scientific) and stained by crystal violet.

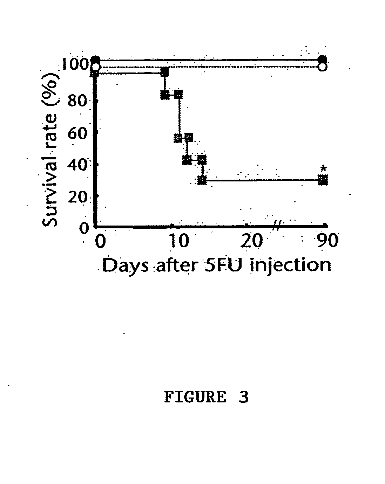

In mice treated with IgG (control mice) and VEGFR-2 MAb-treated mice, the number of leukocytes recovered to base line levels within two weeks after 5FU administration. However, leukocyte counts in mice treated with of anti-VEGFR-1 MAb-treated group failed to recover and 56% died within 3 weeks. Results are shown in Figure 3, which is a graph of survival (%) of the mice as a function of time (days). Moreover, histological analysis of treated and untreated mice demonstrated that BM cellularity were significantly reduced 10 and 20 days after 5FU treatment in VEGFR-1 MAb-treated group.

### Example 7

This example investigates the role of elevated P1GF in ameliorating chemotherapy-induced BM stem cell suppression. Cohorts of mice treated with BM suppressive agents, such as 5FU and carboplatin/radiation, were treated in parallel with and without adenoviral vectors expressing P1GF (AdP1GF, which has been described previously). BALB/c mice received a single i.v. injection of 5FU (300 mg/kg) or carboplatin (1.2 mg) plus total body irradiation (TBI; 5 Gy) on day 0. The number of white blood cells (WBCs) was then determined using standard techniques.

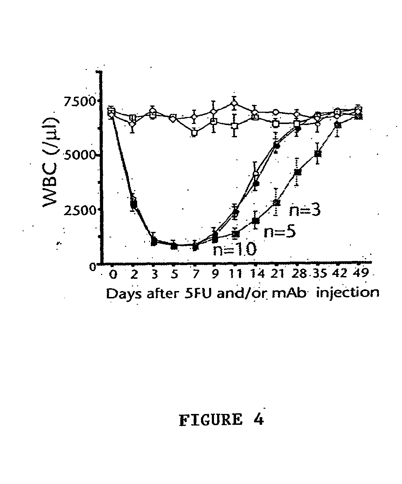

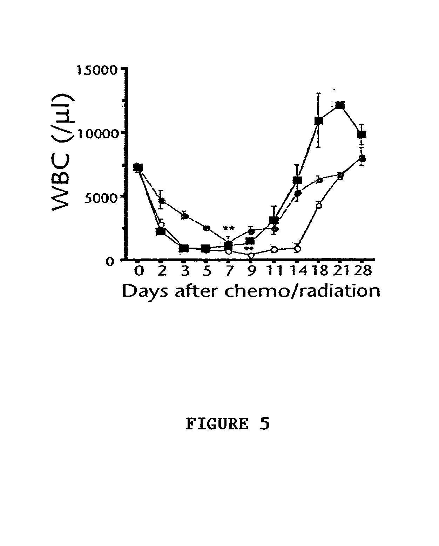

Remarkably, mice that received AdPIGF had significant reduction in extent and duration of neutropenia than control mice. Plasma elevation of P1GF rescued neutropenia similar to standard doses of recombinant granulocyte colony-stimulating factor (G-CSF). Results are shown in Figures 4 and 5, which are graphs of the number of WBC in the mice as a function of time after treatment with either 5FU (Fig. 4) or carboplatin plus TBI (Fig. 5).

Therefore, the present example demonstrates that PIGF, a ligand that binds VEGFR-1, ameliorates the extent and duration of neutropenia after chemotherapy induced bone marrow stem cell supression.

### Example 8

The present example investigates the effect of VEGFR-1 on the motogenic potential of stem cells, particularly HSCs. The capacity of P1GF and VEGF to induce migration of VEGFR-1+CD34⁺ cells were examined in transmigration Boyden Chambers.

A modified version of a previously described transwell migration technique was used. Briefly, LC aliquots (100 µl) were added to 8 µm pore transwell inserts, coated with 25 µg of growth factor-depleted Matrigel (Beckton and Diclanson), and placed into the wells of a 24 well plate. The lower compartment contained serum free RPMI with or without 100 ng/ml P1GF or 50 ng/ml VEGF or 100 ng/ml stromal derived factor-1 (SDF-1) (R&D System). For the purpose of blocking migration, each condition was prepared in a separate aliquot and incubated with anti-VEGFR-1 MAb (clone 6.12; ImClone Inc., 1µg/condition). The migration was carried out at 37° C and 5% CO₂ for 14-18 hrs. Migrated cells were collected from the lower compartment, spun down at 8000 rpm and counted using a hematocytometer. Only live cells, as determined by trypan blue exclusion, were considered in the quantification. Experiments were done in triplicate and results are shown as the number of cells migrated in response to P1GF.

For migration inhibition studies, freshly isolated human CB CD34+ cells were resuspended in serum-free RPMI and a stock of 10⁶ cells/ml was prepared. CD34+ cells were preincubated with 40 µg/ml of MAb to VEGFR-1 in the upper chamber of 6 well transwell plates containing P1GF as indicated. The CB-derived CD34⁺ cells were then placed on upper transmigration chamber of the transwells plate.

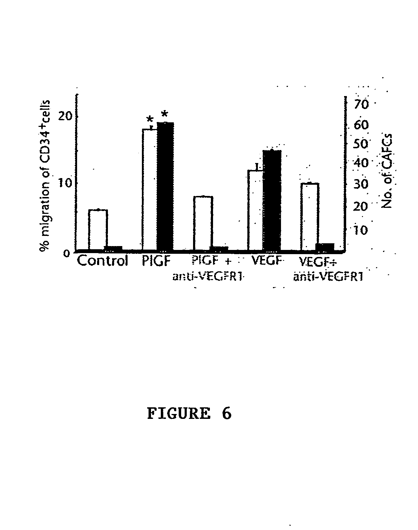

P1GF and VEGF induced migration of 17.0±1.0% and 12.7±0.9% of added CD34⁺ cells to the upper chamber. Neutralizing MAbs to VEGFR-1 inhibited the migration of 8.0±1.2% and 8.5±1.0% of the cells. Results are shown in Figure 6, which is a graph of the number of migrated cells (x 10³ cells) after adminstration of P1GF, P1GF + anti-VEGFR-1 MAb (6.12), VEGF, VEGF + anti-VEGFR-1 MAb, or SDF-1.

These data suggest that the VEGFR-1/P1GF signaling pathway, i.e., the interaction of VEGFR-1 and its ligand, is a potent mediator for the migration of CD34⁺ stem cells.

### Example 9

This example investigates the ability of the VEGFR-1 ligand P1GF to mediate migration of stem cells.

In one experiment, to evaluate the capacity of P1GF to mediate migration of HSCs in a physiological model, P1GF plasma levels were elevated by injecting mice with AdP1GF. Initially in 2- to 3-day intervals and later on a weekly basis, retro-orbital blood was collected with capillary pipettes (Unopette, Fisher Scientific). Total white blood cells and granulocyte (polymorphonuclear leukocytes) were counted using a Neubauer hematocytometer (Fisher Scientific). Differential leukocyte counts were obtained by examination of blood smears from each mouse, stained with Wright-Giemsa stain (200 cells counted/smear). The plasma samples were collected, stored at -80° C and assessed later by immunoassay for human P1GF. Plasma concentration of P1GF were measured using a sensitive ELISA (R&D Systems). PBMCs (10⁴ to 10⁵) were incubated for 30 minutes at 4° C with FITC or PE conjugated MAbs; murine CD11b-PE, Sca-1-PE (PharMingen), VEGFR-1-FTTC (clone MF-1; ImClone Systems). The cells were then analyzed by two-color flow cytometry using a Coulter Elite flow cytometer.

Intravenous administration of AdP1GF resulted in a peak P1GF plasma level (9.5±0.7 ng/ml) 24 hours after injection, and a return to pretreatment level on day 21. AdPIGF-treated mice had a 2-fold increase above baseline in WBC, including monocytic lineage on day 3, returning to the level of AdNull-treated control mice by 4 weeks post injection.

In another experiment, PBMCs were collected from orbital plexus and isolated after centrifugation over a discontinuous gradient using Lympholyte-M (Cederlane). MCs (10⁵ cells) were plated in triplicate in 1 ml of 0.8% methylcellulose containing 30% FCS, 1 % L-glutamine, 2.5% hemin, 0.05 mM 5-ME, IL-3 (50 ng/ml), c-kit ligand (20 ng/ml) and erythropoietin (2 U/ml) in 35-mm suspension culture dishes. Scoring was performed with an inverted microscope with 40 × magnification on day 7. Cells from PBMCs obtained from AdP1GF-treated mice were seeded in the colony assays and four CFU types are scored: CFU-GM, BFU-E, CFU-M and CFU-Mix.

The VEGFR-1 ligand P1GF induced the mobilization of hematopoietic progenitor cells to the peripheral blood of injected mice.

In an additional experiment, the number of P1GF mobilized VEGFR-1⁺Sca-1⁺ cells with stem cell potential, capable of forming spleen colonies (CFU-S), was also measured by injecting P1GF-mobilized PBMCs into lethally (9 Gy) irradiated syngeneic mice. For each data point, three recipient mice were irradiated with 9-Gy from a ¹³⁷Cs γ-ray source to prevent the production of endogenous spleen colonies. Irradiated BALB/c mice (three mice in each group) were injected i.v. via the tail vein with 1 × 10⁵ PBMCs within several hours after the completion of irradiation. The mice were sacrificed by cervical dislocation 12 days later, and their spleens were removed and fixed in Bouin's solution. The number of macroscopic spleen colonies was then scored.

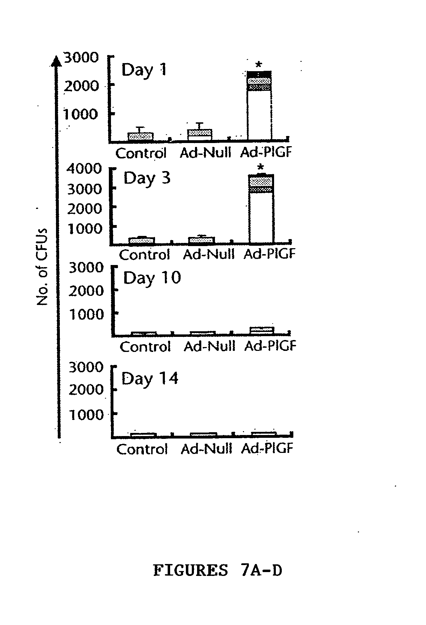

Administration of AdP1GF induced a 20-fold increase in the mobilization of CFU-S to the peripheral blood by day 3 of treatment. Results are shown in Figures 7A-D, which are graphs of the number of colonies after administration of P1GF as compared to various controls at day 1 (Fig. 7A), day 3 (Fig. 7B), day 10 (Fig. 7C), and day 14 (Fig. 7D).

Finally, the number of mobilized pluripotent hematopoietic cells with BM-repopulating capacity was also determined by transplantation of AdP1GF mobilized PBMC into lethally irradiated syngeneic mice. After adenoviral vector administration, the peripheral blood from BALB/c mice (9 mice in each group) was collected on day 5 using Lympholyte-M to remove erythrocytes. The recipient BALB/c mice (6 mice in each group) were lethally irradiated (9 Gy) and i.v. injected with serial cell doses (5 × 10⁴, 1 × 10⁵, 5 × 10⁵ and 1×10⁶) PBMCs after irradiation on day 0.

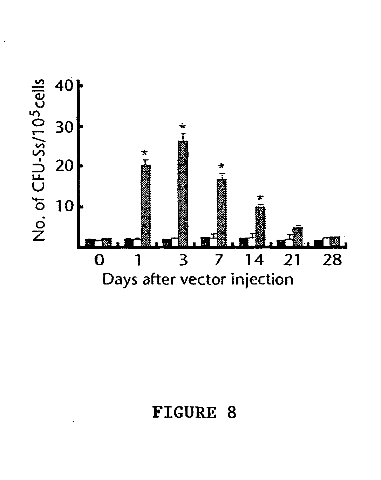

Injection of AdP1GF, but not AdNull, resulted in mobilization of HSC that were able to engraft and rescue lethally irradiated mice. Results are shown in Figure 8, which is a graph of the number of CFU-S colonies as a function of the number of days after adenoviral vector administration.

These data suggest that activation of VEGFR-1 on stem cells, i.e., HSCs, regulate the motogenic potential rather than survival or proliferation of HSCs.

### Example 10

The present example investigates the mechanism by which VEGFR-1 affects BM hematopoiesis.

In one experiment, the capacity of neutralizing MAb to VEGFR-1 to block cycling potential of HSCs after 5FU treatment was investigated. All BALB/c mice were injected i.v. with 5FU (300 mg/kg) via tail vein. Eight 5FU-treated mice in each group injected i.p. with 800 µg of anti-VEGFR-1 (clone MF-1, ImClone Systems) or human IgG at 2 days interval from either day 0. We monitored leukocyte count at 2 to 3 days interval for 30 days and at least 2 mice in each group were sacrificed by cervical dislocation at each time point. BMMC were harvested from mice in each group and fixed in cold ethanol (4 °C) for 1 hour. Then, cells were treated with RNAse (Sigma) at room temperature (20 °C) for 5 minute and stained with propidium iodide (Molecular Probes). The DNA content was determined by flow cytometric analysis. Each analysis was done at least twice.

5FU treated mice that received anti-VEGFR-1 MAb showed no evidence of cycling Sca-1 in the BM (Fig. 5). These data set forth the hypothesis that one mechanism that VEGFR-1 activation can promote hematopoiesis is through induction of cycling of HSCs.

In another experiment, the activation of matrix metalloproteinase (MMP) -9 was evaluated. Supernatants from human CD34⁺ cell cultures were collected after overnight incubation in serum-free medium, with or without P1GF or VEGF or SDF1, and their MMP-9 activity was measured by gelatinolytic zymography, as previously described (Huang et al., Biochem. Biophys. Res. Commun. 264:133-8 (1999)). Briefly, cell culture supernatants were treated with gelatin-agarose beads, to concentrate the gelatinases, and processed through SDS-Page-acrylamide gels containing 1 % gelatin. The gels were subsequently incubated in 2.5% Triton X-100 for 1 hr at room temperature (RT), rinsed in distilled water (DW) and placed in low-salt collagenase buffer (50 mM Tris- pH 7.6, 0.2 M NaCl, 5mM CaCl₂ and 0.2% v/v Brij-35) at 37° C for 18hrs. Bands of gelatinolytic activity were visualized after staining the gels with 10 mL of a 0.2% Coomassie blue solution and 190 mL destain (DW, Methanol and glacial acetic acid, 6:3:1) for 30 mins-1hr at RT. For each experiment, supernatants from 1×10⁶ cells were collected and each experiment was done in triplicate. The Adobe Photoshop 4.0 software application and a Umax Astra scanner were used to scan the gels and the intensity of the gelatinolytic bands was assessed using NIH Image 1.58. Total WBCs were also determined.

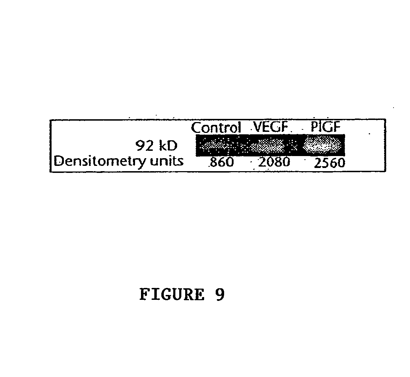

Elevation of P1GF results in induction of MMP-9 activation in human CD34⁺ cells. Results are shown in Figure 9, which is a graph of the densinometric intensity of the gelatinolytic bands after administration of SDF-1, VEGF, or P1GF. Conversely, elevation of P1GF fails to mobilize HSCs in MMP-9 deficient mice. MMP-9 is necessary for cycling of HSCs and activation results in the release soluble kit-ligand that promotes entry of HSCs into cell cycle.

Collectively, this example suggests that VEGFR-1 activation, particularly through its ligand P1GF, is one of the critical pathways that promote cycling of stem cells, e.g., HSCs involving MMP-9 activation.

## Claims

1. Use of mammalian stem cells expressing VEGFR-1 or of compositions comprising mammalian stem cells expressing VEGFR-1 for preparing pharmaceutical compositions for the induction of hematopoiesis, vasculogenesis, angiogenesis, myogenesis, neurogenesis, mobilization of stem cells to the area of administration or for reconstituting pancreatic islet cells, regenerating damaged neurons or destructed bone marrow or for the treatment of cardiac or peripheral ischemia,
wherein the stem cells are no human embryonic stem cells, and are obtainable by
- providing a population of cells,
- contacting the population of cells with a molecule that specifically binds to VEGFR-1, and
- isolating cells that bind the molecule that specifically binds to VEGFR-1.

2. Use according to claim 1, wherein the molecule that specifically binds to VEGFR-1 is an antibody.

3. Use according to claim 1, wherein the molecule that specifically binds to VEGFR-1 is a ligand.

4. Use according to claim 1 or 3, wherein the molecule that specifically binds to VEGFR-1 is PIGF.

5. Use according to any of claims 1 to 4, wherein the population of cells is isolated from a fetal liver, umbilical cord blood, a yolk sac, a mature spinal cord, bone marrow, an adult peripheral blood sample or from the central nervous system.

## Patentansprüche

1. Verwendung von Säuger-Stammzellen, die VEGFR-1 exprimieren, oder von Zusammensetzungen, die Säuger-Stammzellen enthalten, die VEGFR-1 exprimieren, zur Herstellung von pharmazeutischen Zusammensetzungen zur Induktion von Hämatopoese, Vasculogenese, Angiogenese, Myogenese und Neurogenese, zur Mobilisierung von Stammzellen zum Gebiet der Verabreichung oder zur Wiederherstellung von Pankreas-Inselzellen, zur Regeneration beschädigter Neuronen oder von zerstörtem Knochenmark oder zur Behandlung von cardialer oder peripherer Ischämie,
wobei die Stammzellen keine menschlichen embryonalen Stammzellen sind und erhältlich sind durch
- Bereitstellen einer Population von Zellen,
- Inkontaktbringen der Population von Zellen mit einem Molekül, das spezifisch an VEGFR-1 bindet, und
- Isolieren von Zellen, die das Molekül binden, das spezifisch an VEGFR-1 bindet.

2. Verwendung nach Anspruch 1, wobei das Molekül, das spezifisch an VEGFR-1 bindet, ein Antikörper ist.

3. Verwendung nach Anspruch 1, wobei das Molekül, das spezifisch an VEGFR-1 bindet, ein Ligand ist.

4. Verwendung nach Anspruch 1 oder 3, wobei das Molekül, das spezifisch an VEGFR-1 bindet, PIGF ist.

5. Verwendung nach einem der Ansprüche 1 bis 4, wobei die Population von Zellen isoliert ist aus einer fötalen Leber, Nabelschnurblut, einem Dottersack, einem reifen Rückenmark, Knochenmark, einer Probe von peripherem Blut eines Erwachsenen oder aus dem Zentralnervensystem.

## Revendications

1. Utilisation médicale de cellules souches mammifères exprimant VEGFR-1 ou de compositions comprenant des cellules souches mammifères exprimant VEGFR-1 pour la préparation de compositions pharmaceutiques pour l'induction de hématopoïèses, de vasculogenèses, d'angiogenèses, de myogenèses, de neurogenèses, pour la mobilisation de cellules souches vers la zone d'administration ou pour la reconstitution de cellules pancréatiques insulaires, la régénération de neurones lésés ou de la moelle osseuse ou pour le traitement de maladies cardiaques ou périphériques ischémiques,
dans laquelle les cellules souches ne sont pas des cellules souches embryonnaires humaines, et qu'elles sont obtenues par
- la production d'une population de cellules,
- la mise en contact de la population de cellules avec une molécule qui spécifiquement se lie à VEGFR-1, et
- l'isolation de cellules qui lient la molécule qui spécifiquement se lie à VEGFR-1.

2. Utilisation selon la revendication 1, dans laquelle la molécule qui spécifiquement se lie à VEGFR-1 est un anticorps.

3. Utilisation selon la revendication 1, dans laquelle la molécule qui spécifiquement se lie à VEGFR-1 est un ligand.

4. Utilisation selon l'une quelconque des revendications 1 à 3, dans laquelle la molécule qui spécifiquement se lie à VEGFR-1 est le PIGF.

5. Utilisation selon l'une quelconque des revendications 1 à 4, dans laquelle la population de cellules est isolée à partir d'un foie foetal, de sang d'un cordon ombilical, d'une membrane vitelline, d'un cordon spinal à l'état de maturité, de la moelle osseuse, d'un échantillon de sang périphérique d'un adulte ou du système nerveux central.
